(19) 

**Europäisches Patentamt**
**European Patent Office**
**Office européen des brevets**

(11) **EP 3 894 855 B1**

(12) **EUROPEAN PATENT SPECIFICATION**

(45) Date of publication and mention
of the grant of the patent:
**03.09.2025 Bulletin 2025/36**

(21) Application number: **19845823.4**

(22) Date of filing: **16.12.2019**

(51) International Patent Classification (IPC):
**G01N 33/543** (2006.01)

(52) Cooperative Patent Classification (CPC):
**G01N 33/54306; G01N 33/54313; G16C 20/20;**
G16B 40/10; G16C 20/70

(86) International application number:
**PCT/GB2019/053566**

(87) International publication number:
**WO 2020/120996 (18.06.2020 Gazette 2020/25)**

(54) **ASSAY ANALYSIS**

TESTANALYSE

ANALYSE DE DOSAGE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **14.12.2018 GB 201820395**

(43) Date of publication of application:
**20.10.2021 Bulletin 2021/42**

(73) Proprietor: **Aeirtec Ltd**
**North Shields, Tyne and Wear NE30 1JE (GB)**

(72) Inventor: **KILFEATHER, Stephen**
**Clifford Ford**
**North Shields Tyne and Wear NE30 1JE (GB)**

(74) Representative: **Hartley, Lucinda Jane**
**Hargreaves Elsworth**
**26 Northumberland Square**
**North Shields, Tyne and Wear NE30 1PW (GB)**

(56) References cited:
**WO-A1-2012/087840          WO-A1-2017/032848**
**US-A1- 2014 256 588**

• **MIKAEL KARLSSON: "Determination of antibody**
**affinity and kinetic binding constants in Gyrolab**
**Bioaffy microfluidic CD", 1 January 2008**
**(2008-01-01), XP055019743, Retrieved from the**
**Internet <URL:liu.diva-portal.org/smash/get/**
**diva2:18035/FULLTEXT01> [retrieved on**
**20120217]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 3 894 855 B1

## Description

### Field of the Invention

The present invention relates to systems and methods for computer-aided quantification of soluble analytes in fluids.

### Background of the Invention

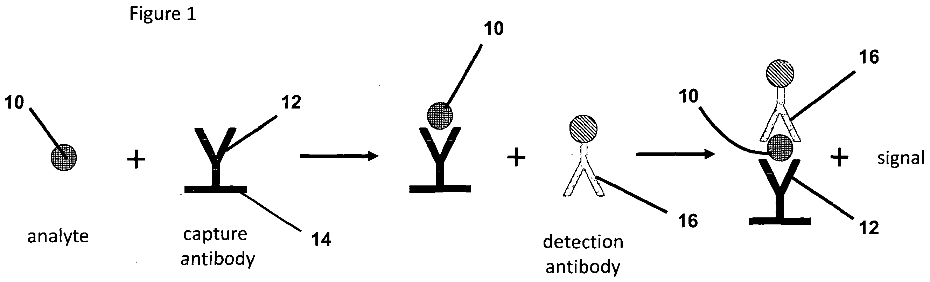

Quantification of soluble analytes occurs frequently in many biological, medical, research, industrial, food production and security fields. Analytes can include, for example, antibodies, other proteins, peptides, medicinal molecules (small and large) and hormones. The exploitation of the affinity and specificity of protein molecules called 'antibodies', generated in immune systems, in quantification of soluble analytes in 'immunoassay' began in the 1950's with measurement of insulin in blood plasma samples (Yalow RS, Berson SA. Assay of plasma insulin in human subjects by immunological methods. Nature. 1959;184 (Suppl 21):1648-1649). The dependence on radioactive signalling for analyte detection was replaced by the use of enzyme-based colour generation as a route for signal amplification. This gave rise to the generation of enzyme immunoassays (EIA) (Van Weemen BK, Schuurs AHWM. Immunoassay using antigen-enzyme conjugates. FEBS Lett. 1971;15(3):232-236) and enzyme-linked immunosorbent assay (ELISA) (Engvall E, Perlmann P. Enzyme-linked immunosorbent assay (ELISA): quantitative assay of immunoglobulin G. Immunochemistry.1971;8(9):871-874). These common forms of assay system incorporate molecules or antibodies that bind to (or 'capture') analyte molecules prior to their quantification in an assay. Capture molecules are themselves most often immobilised by attachment to a generally solid platform ('substrate'). The substrate is commonly a flat plane material or 'bead-like' particle. In one such common assay form, termed a 'sandwich immunoassay', illustrated in Figure 1, a capture antibody molecule 12 is bound to a substrate 14. The capture of the analyte 10 by the capturing antibody molecule 12 is then revealed by binding of a different antibody ('detection antibody', 16) to the analyte 10. Signalling moieties already attached to the detecting antibody molecule 16 provide the indication that analyte is present on the capture molecule. Thus, the signal generation indicates binding of detection antibody and therefore analyte presence. Signalling moieties attached to the detecting antibody molecule 16 commonly include fluorescent or chemiluminescent compounds so that the signal produced can be detected using cameras or fluorimeters, or enzymes for induction of colour change in the locality of the enzyme so that colour can be detected by light absorbance. The signalling moiety may also initiate an electrochemical reaction also detected by change in colour or emission.

Apparatus and methods for conducting solid-phase binding assays is described in US2014/256588, WO2017/032848, and Mikael Karlsson (Master Thesis, Linköping University, 01.04.2008): "Determination of antibody affinity and kinetic binding constatis in Gyrolab Bioaffy microfluidic CD". WO2012/087840 describes injection methods for biosensing applications which can be used to determine injected analyte concentration.

Where analytes are relatively small, often a 'competitive' assay format is generated in which the interaction of analyte with capture antibody is detected through its capacity to compete with another molecule ('non-analyte competing molecule') for interaction with the capture antibody. The non-analyte competing molecule in this case can itself be the source of detection signal through detection moieties attached to the non-analyte competing molecule.

Alternative forms of immunoassay include the proximity-dependent assays. In one example, the capture antibody is attached to a first type of bead and detection antibody is attached to a second type of bead. The first type of bead emits a first type of excitation emission that excites a second type of emission from the second type of bead. Thus, emission from the second type of bead requires close proximity of the first type of bead and therefore is dependent upon the interaction between capture and analyte and simultaneous interaction between detection antibody and analyte. Such a system has been demonstrated by PerkinElmer in the Alphalisa system. Another example of proximity-dependent assay is the proximity ligation assay in which differing species-derived detection antibodies bind to an immobilised analyte at differing loci and secondary antibodies separately recognising the different species detection antibody constant regions bind to the primary antibodies. The secondary antibodies have a unique short strand DNA attached and the DNA strands can engage in a rolling circle DNA synthesis as an amplifying signal generation.

Despite the introduction of fluorescence and chemiluminescence as alternative signalling systems, assay sensitivity remains a significant limiting factor in utilization of immunoassay. Many analyte molecules exist at relatively low levels that are not always measurable with existing assay technology capacity and their quantification is therefore limited by the sensitivity of the measuring system employed. Insufficient assay sensitivity has therefore emerged as one of the most significant 'problems' in soluble analyte immunoassay (J. Comley, ELISA Assays: recent innovations take analyte detection to new levels. Drug Discovery World, 2012). As an example, top of the hierarchy immune/inflammatory reaction initiating molecules exist at relatively low levels. Within this group of analytes, tumor necrosis factor (TNF) functions at concentrations as low as femtogram per milliliter (fg/ml) in circulating blood. The importance of TNF in driving immune disease and importance of monitoring its level are reflected by the presence of three different TNF-inhibiting drugs within the top ten selling drugs in 2017.

Therefore, in health-related biology alone, the capacity to monitor the activity of low abundance, but powerful molecules involved in disease generation and maintenance, is essential to understanding physiology, monitoring of disease, development of therapeutic agents and monitoring of medicinal therapeutic efficacy.

When carrying out immunoassay for quantification of soluble analyte it is necessary to construct a calibration curve, often termed a 'standard curve' by plotting a series of known concentrations of an analyte against the signal intensity results. For noncompetitive immunoassays this results in plots that have the lowest level of signal at the lowest analyte concentrations with progressive increase in signal to the highest analyte concentrations. Whereas in competitive assays, the shape of the noncompetitive plot is mirrored and the highest signals are generated at the lowest analyte concentrations with progressive decrease in signal as the analyte concentrations increase. Competitive assay plots can be converted to the same format as noncompetitive plots by plotting the degree of inhibition of the non-competing molecule binding to capture antibody, giving the lowest inhibition values at the lowest analyte concentrations with a progressive rise in degree of inhibition with increasing analyte concentration. This provides a convenient way of applying some equations for standard curve fitting wherein the values of signal in the equation are replaced with values of degree of inhibition of signal generation.

The standard curve plot is mathematically modelled with an equation to be used in quantifying the analyte concentrations of unknown samples. The type of curve-fitting model used, along with the fit of the model to the results has a direct effect on the accuracy of the results and sensitivity of detection and quantification.

Absorbance of colour is not linearly related to coloured molecule concentration, with colour generation forming a rectangular hyperbola when plotted against colour concentration. Therefore in enzyme-based colour generation, as in enzyme-amplification as signal source in ELISA, the distribution of plotted data points within a standard curve can be practically linear when colour absorbance as signal is plotted against the log of analyte concentration. Antibody occupancy by antigen analyte also forms a rectangular hyperbola with analyte concentration. With the introduction of more sensitive signalling systems to ELISA systems, such as fluorescence and chemiluminescence, these systems enabled quantification of analyte over larger concentration ranges (assay 'dynamic range'). With increasing dynamic range it is more practical to format the analyte concentration axis in logarithm (log) form. Thus, assay dynamic ranges of around three to four log units produce sigmoidal curves between signal and log of analyte concentration.

Analysis of sigmoid curves to generate a relationship between signal and analyte concentration requires logistic regression for curve fitting and subsequent interpolation of signals derived from tested samples for quantification of analyte within. Two logistic equation models are ubiquitous in use to model the behaviour of analyte-capture antibody interaction in sigmoidal curve fitting: the four parameter logistic equation (4-PL) and five parameter logistic equation (5-PL).

Four Parameter Logistic equation model (4-PL)

The 4-PL equation contains four parameters related to the graphical properties of the curve. (G. M. Raab. Comparison of a Logistic and a Mass-Action Curve for Radioimmunoassay Data. Clin. Chem. CLIN. CHEM. 29(10), 1757-1761. 1983; D. Finney. Response Curves for Radioimmunoassay. Clin. Chem., 29(10), 1762-1766, 1983; R. A. Dudley, P. Edwards, R. P. Ekins, D. J. Finney, I. G. M. McKenzie, G. M. Raab, D. Rodbard, and R. P. C. Rodgers. Guidelines for Immunoassay Data Processing. Clin. Chem., 31(8) ,1264-1271, 1985; P Strohner, D Sarrach, and J G. Reich. Use of a modified binding model for the investigation of affinity dependence on antibody concentration in immunoassay systems. Journal of Immunological Methods 203,113-122, 1997; P. G. Gottschalk, J. R. Dunn. The five-parameter logistic: A characterization and comparison with the four-parameter logistic. Analytical Biochemistry 343, 54-65, 2005).

The equation is as follows:

$$y = d + \frac{a\text{-}d}{1 + (x/c)^b}$$

where:y = the response or signal

d = estimated response at infinite analyte concentration

a = estimated response at zero analyte concentration

x = analyte concentration

c = the inflection point on the calibration curve indicating the concentration of analyte predicted to produce 50% of maximum signal (equivalent to the dissociation constant Kd in binding studies)

b = slope factor that defines the curve slope.

Once a 4-PL equation is created from a set of data, values for all four parameters are determined and the equation can then be used to calculated unknown concentrations (x) from the assay signal data (y).

Five Parameter Logistic equation model (5-PL)

Immunoassay standard curves for quantification of soluble analyte can be asymmetric and the 5-PL equation is often employed in an attempt to provide a better fit. The 5-PL equation includes an additional parameter for asymmetry.

The equation is as follows:

$$y = d \ + \ \frac{a\text{-}d}{[1 + (x/c)^b]^g}$$

where: y = the response

d = estimated response at infinite analyte concentration
a = estimated response at zero analyte concentration
x = analyte concentration
c = the inflection point on the calibration curve indicting the concentration of analyte predicted to produce 50% of maximum detection signal (equivalent to the dissociation constant Kd in binding studies)
b = slope factor that defines the curve slope.
g = asymmetry factor

Invariably, the 4-PL and 5-PL are applied with a range of optional weightings to vary the level of influence points in the lower or upper region have on the position of the curve. $1/Y^2$ is often provided as a default weighting option.

The type of logistic equation which yields the best fit through a set of data points is dependent on the shape of the standard curve of an assay. However, neither the 4-PL, nor the 5-PL equation gives a good fit in relation to low concentration regions of many soluble analyte immunoassays. Many standard curves generated with these two equations are truncated prematurely in their lower regions due to the unreliability of curve fitting in the lower concentration ranges. Independent of a decreased signal to noise ratio at low analyte concentrations, the poor fitting of 4-PL and 5-PL curve fitting could be due to assumptions in the models that they provide, constraining the curve fitting away from properly fitting the lower section of sigmoidal immunoassay curves.

Some new technologies have been designed to improve analyte detection at low concentration levels. However, some of these technologies focus only on relatively low concentration ranges and in some cases have a relatively low sample throughput (Quanterix Simoa technology), or low throughput in terms of being able to measure more than one analyte simultaneously within a single assay chamber ('Multiplexing')( Singulex, Chimera).

It would be desirable to provide an improved method for quantification of soluble analytes in fluids, particularly at low concentrations, while maintaining dynamic range. Improvement of systems incorporating modelling of the relationship between analyte concentration and signal generation are required to enable further improvement in assay sensitivity while maintaining or extending assay dynamic range.

**Summary of the Invention**

According to a first aspect of the invention there is provided a method for determining the concentration of an analyte in a sample comprising the steps of:

(a) providing a plurality of samples of the analyte at known concentrations;
(b) providing analyte capture molecules bound to a solid substrate, and detection molecules, the analyte capture molecules and detection molecules both suitable for binding with the analyte, wherein the detection molecules include signalling means to identify a reaction with the analyte;
(c) mixing the samples of analyte with the analyte capture molecules and detection molecules to form an analyte-capture molecule-detection molecule complex;
(d) detecting a signal resulting from the reaction of the analyte with the detection molecules;
(e) applying a mathematical transformation function to the detected signal;
(f) using a computer to mathematically model a biphasic standard curve of analyte concentration vs transformed detected signal of step (e) using the equation:

$$fY = \frac{fBmax1 \times [L]}{Kd1 + [L]} + \frac{fBmax2 \times [L]}{Kd2 + [L]}$$

where $f$ is a mathematical transformation function used in step (e), which amplifies the signal values at lower concentrations relative to the higher concentrations within the assay range;

Y is the signal generated at an analyte concentration [L];

Bmax1 is the maximal signal generated from a higher affinity analyte-capture molecule interaction;

Kd1 is the dissociation constant of the higher affinity analyte-capture molecule interaction;

Bmax2 is the maximal signal generated from a lower affinity analyte-capture molecule interaction; and

Kd2 is the dissociation constant of the lower affinity analyte-capture molecule interaction;

(g) providing a sample of the analyte of unknown concentration;

(h) providing analyte capture molecules and detection molecules, the analyte capture molecules and detection molecules both suitable for binding with the analyte, wherein the detection molecules include signalling means to identify a reaction with the analyte;

(i) mixing the samples of analyte with the analyte capture molecules and detection molecules to form an analyte-capture molecule-detection molecule complex;

(j) detecting a signal resulting from the reaction of the analyte with the detection molecules;

(k) applying a mathematical transformation function to the detected signal of step (j); and

(l) determining the concentration of the analyte in the sample by correlating the transformed detected signal of step (k) with the analyte concentration using the biphasic standard curve generated in step (f).

A further aspect of the invention provides a method for providing a calibration graph for an assay comprising the steps of:

(a) providing a plurality of samples of an analyte at known concentrations;

(b) providing analyte capture molecules and detection molecules, the analyte capture molecules and detection molecules both suitable for binding with the analyte, wherein the detection molecules include signalling means to identify a reaction with the analyte ;

(c) mixing the samples of analyte with the analyte capture molecules and detection molecules to form an analyte-capture molecule-detection molecule complex;

(d) detecting a signal resulting from the reaction of the analyte with the detection molecules;

(e) applying a mathematical transformation function to the detected signal;

(f) using a computer to plot a graph of the known analyte concentration vs the transformed detected signal from step (e) and to mathematically model a biphasic standard curve of analyte concentration vs transformed detected signal of step (e) using the equation:

$$fY = \frac{fBmax1 \times [L]}{Kd1 + [L]} + \frac{fBmax2 \times [L]}{Kd2 + [L]}$$

where $f$ is a mathematical transformation function used in step (e), which amplifies the signal values at lower concentrations relative to the higher concentrations within the assay range;

Y is the signal generated at an analyte concentration [L];

Bmax1 is the maximal signal generated from a higher affinity analyte-capture molecule interaction;

Kd1 is the dissociation constant of the higher affinity analyte-capture molecule interaction;

Bmax2 is the maximal signal generated from a lower affinity analyte-capture molecule interaction; and

Kd2 is the dissociation constant of the lower affinity analyte-capture molecule interaction.

A further aspect of the invention is a computer as defined in claim 11.

A further aspect of the disclosure provides a computer programmed to mathematically model a biphasic standard curve of analyte concentration vs transformed detected signal using the equation

$$fY = \frac{fBmax1 \times [L]}{Kd1 + [L]} + \frac{fBmax2 \times [L]}{Kd2 + [L]}$$

where *f* is a mathematical transformation function which amplifies the signal values at lower concentrations relative to the higher concentrations within the assay range, as hereinbefore defined;

Y is the signal generated at an analyte concentration [L];

Bmax1 is the maximal signal generated from a higher affinity analyte-capture molecule interaction;

Kd1 is the dissociation constant of the higher affinity analyte-capture molecule interaction;

Bmax2 is the maximal signal generated from a lower affinity analyte-capture molecule interaction; and

Kd2 is the dissociation constant of the lower affinity analyte-capture molecule interaction.

An assay is a procedure for the detection or quantification of an analyte. The assay may be an immunoassay. The assay is preferably a sandwich-type assay. The mathematical transformation function *f* amplifies the signal values at lower concentrations relative to the higher concentrations within the assay range. Any mathematical transformation that achieves this result may be used. The transformation function may be selected from the group comprising: $\log_n x$, ln $x$, ASINH(x), and $\sqrt[n]{x}$ or $x^{\frac{1}{n}}$, where $x$ = signal results to be transformed an *n* is between 2 and 100. Preferably n is between 5 and 10, and more preferably n=10.

The equation in step (f) may further include a term relating to background signalling, Bkd, at analyte concentration of zero:

$$fY = \frac{fBmax1 \times [L]}{Kd1 + [L]} + \frac{fBmax2 \times [L]}{Kd2 + [L]} + fBkd$$

Bkd is the 'background' signal obtained when [L] = 0 (zero).

Where the background (Bkd) signal value is subtracted from the total signal data before transformation and standard curve generation, or its influence not modelled in the computation, then the fBkd term is removed from the equation.

The equation in step (f) may further include a term relating to nonspecific signalling, NS, which varies with analyte concentration, f(NS x [L]):

$$fY = \frac{fBmax1 \times [L]}{Kd1 + [L]} + \frac{fBmax2 \times [L]}{Kd2 + [L]} f(NS \times [L])$$

NS is the slope of change in signal derived from nonspecific binding of analyte with [L].

Where the signal values derived from nonspecific binding of analyte (NS x [L]) are subtracted from the total signal data before transformation and standard curve generation, or its influence not modelled in the computation, then the f(NS x [L]) term is removed from the equation.

The equation in step (f) may include a term relating to background signalling, Bkd, at analyte concentration of zero, and a term relating to nonspecific signalling, NS, which varies with analyte concentration, f(NS x [L]):

$$fY = \frac{fBmax1 \times [L]}{Kd1 + [L]} + \frac{fBmax2 \times [L]}{Kd2 + [L]} + f(NS \times [L]) + fBkd$$

Preferably, the number of samples of known concentration in step (a) is at least six. More preferably, the number of samples of known concentration in step (a) is at least seven.

The signalling means of the detection molecule or, as an example antibody, may be selected from the group comprising colour-generating labels, chemiluminescent labels, fluorescent labels, phosphorescent labels, bioluminescent labels, electrochemiluminescent labels, crystalloluminescent labels, incandescent labels or radiation labels.

In steps (d) and (j) of either method, the signal may be detected using a variety of imaging devices, for example x-ray detectors, alpha, beta and gamma radiation detectors, CCD camera imaging devices, CMOS camera imaging devices, phosphorimagers, fluorimeters, flow cytometers, time resolved fluorescence spectrometers, fluorescence polarization analysers, quantitative polymerisation chain reaction reporters and spectrophotometers. The signal may be detected using X-ray film. Chemiluminescence may be detected using phosphorimagers. Ultraviolet (UV) and visible light (colours) may be detected through absorbance with a spectrophotometer or a UV/colour-sensitive CCD or CMOS camera.

The equation may further include a term relating to background signalling, Bkd, at analyte concentration of zero:

$$fY = \frac{fBmax1 \times [L]}{Kd1 + [L]} + \frac{fBmax2 \times [L]}{Kd2 + [L]} + fBkd$$

The equation may further include a term relating to nonspecific signalling, NS, which varies with analyte concentration, f(NS x [L]):

$$fY = \frac{fBmax1 \times [L]}{Kd1 + [L]} + \frac{fBmax2 \times [L]}{Kd2 + [L]} \, f(NS \times [L])$$

As hereinbefore described, the equation may include a term relating to background signalling, Bkd, at analyte concentration of zero, and a term relating to nonspecific signalling, NS, which varies with analyte concentration, f(NS x [L]):

$$fY = \frac{fBmax1 \times [L]}{Kd1 + [L]} + \frac{fBmax2 \times [L]}{Kd2 + [L]} + f(NS \times [L]) + fBkd$$

A further aspect of the disclosure provides a kit of parts for determining the amount of an analyte in a sample comprising:

at least one assay substrate coated with an analyte capture molecule suitable for binding with the analyte;
at least one detection molecule suitable for binding with the analyte, the detection molecule including signalling means to identify a reaction with the analyte; and
a set of instructions for modelling calibration data for an assay comprising the steps of:

(i) Adding a plurality of samples of the analyte at known concentrations to the assay substrate;
(ii) Adding the detection molecules to the assay substrate;
(iii) Detecting a signal resulting from the reaction of the analyte with the detection molecules;
(iv) applying a mathematical transformation function to the detected signal; and
(v) using a computer to mathematically model a biphasic standard curve of analyte concentration vs transformed detected signal of step (e) using the equation:

$$fY = \frac{fBmax1 \times [L]}{Kd1 + [L]} + \frac{fBmax2 \times [L]}{Kd2 + [L]}$$

where $f$ is a mathematical transformation function which amplifies the signal values at lower concentrations relative to the higher concentrations within the assay range;
Y is the signal generated at an analyte concentration [L];
Bmax1 is the maximal signal generated from a higher affinity analyte-capture molecule interaction;
Kd1 is the dissociation constant of the higher affinity analyte-capture molecule interaction;
Bmax2 is the maximal signal generated from a lower affinity analyte-capture molecule interaction; and
Kd2 is the dissociation constant of the lower affinity analyte-capture molecule interaction.

The at least one assay substrate in the kit may be in the form of a multi-well plate. Alternatively, the at least one assay substrate may be in the form of a particle or bead.
The detection molecule may be provided as a concentrated solution, or in freeze-dried (lyophilised) form.
The kit of parts may further comprise at least one calibration analyte sample. The calibration analyte sample is an analyte sample used to make the analyte samples of known concentration in step (i). The calibration analyte sample may be provided as a concentrated solution, or in freeze-dried (lyophilised) form.
The invention provides an improved method for quantification of soluble analytes in fluids. The invention allows an assay, such as an immunoassay to be carried out with increased dynamic and Lower Limit of Quantification (LLOQ) compared with prior art methods.

**Brief Description of the Drawings**

In the Drawings:

Figure 1 illustrates a sandwich immunoassay;

Figures 2 to 11 illustrate a series of standard curves plotted in relation to immunoassay of ten different example analytes; comparing biphasic curve modelling to 5-PL curve modelling and two-site curve modelling;

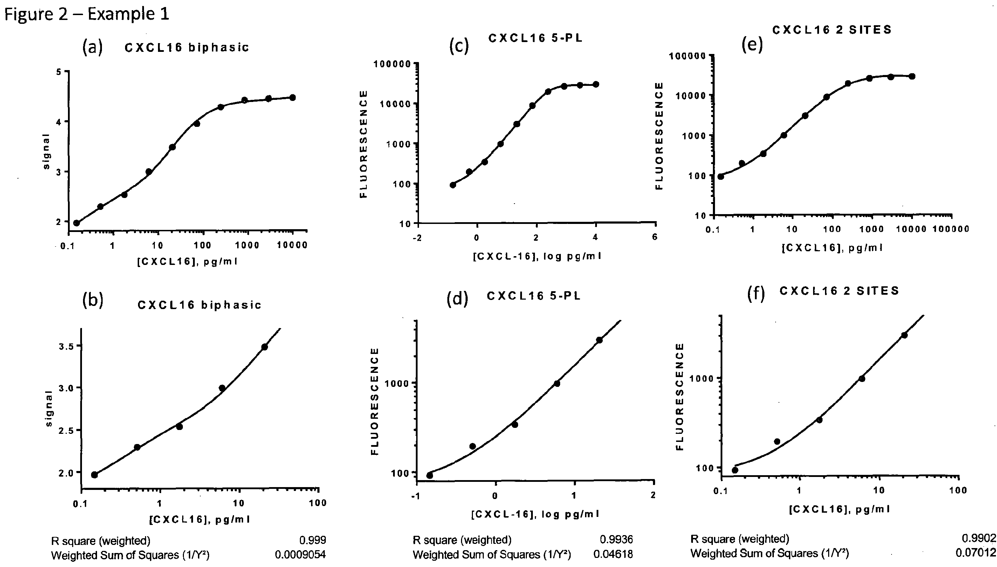

Figure 2 illustrates the standard curves of the analyte CXCL16;

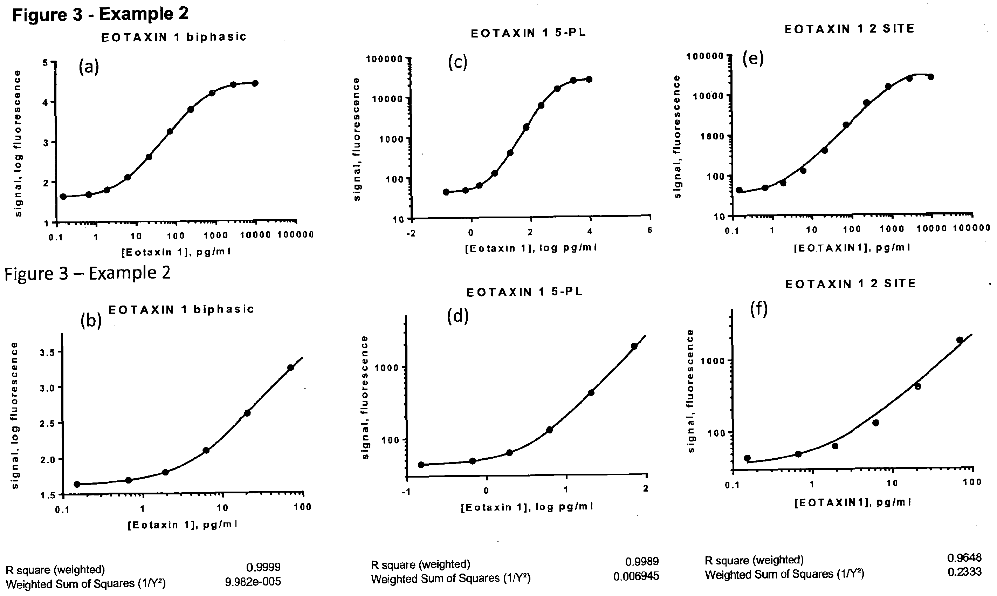

Figure 3 illustrates the standard curves of the analyte EOTAXIN 1;

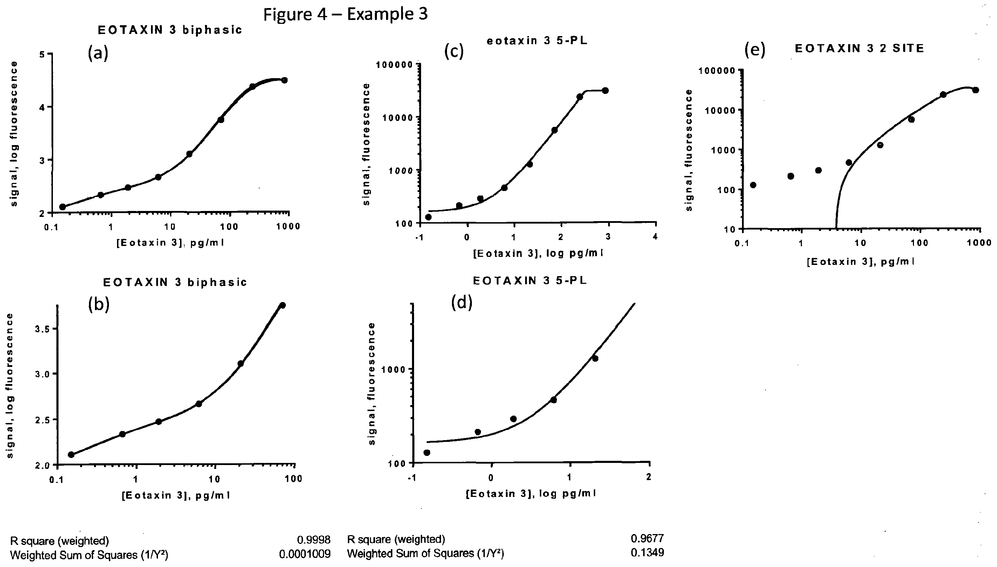

Figure 4 illustrates the standard curves of the analyte EOTAXIN 3;

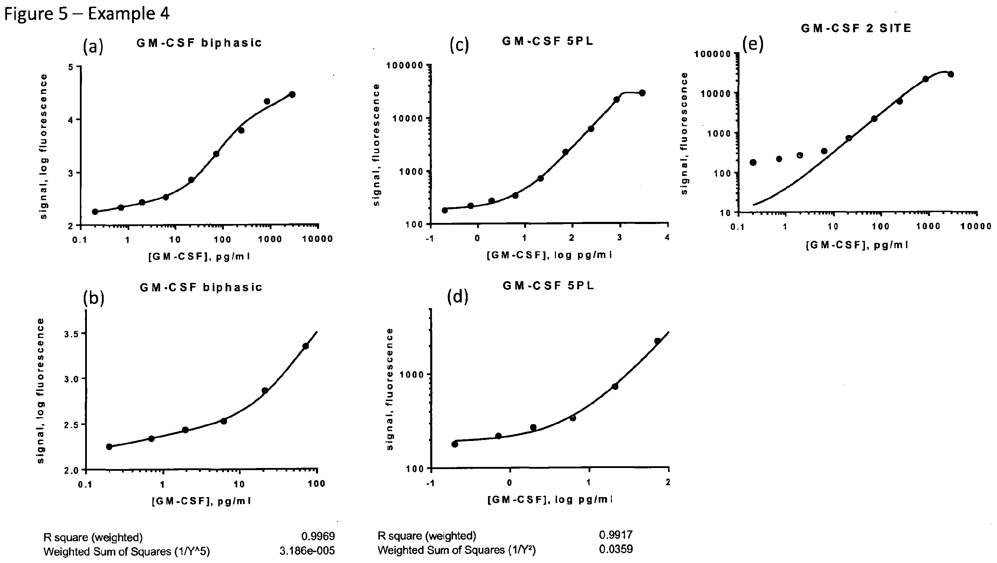

Figure 5 illustrates the standard curves of the analyte granulocyte macrophage -colony stimulating factor (GM-CSF);

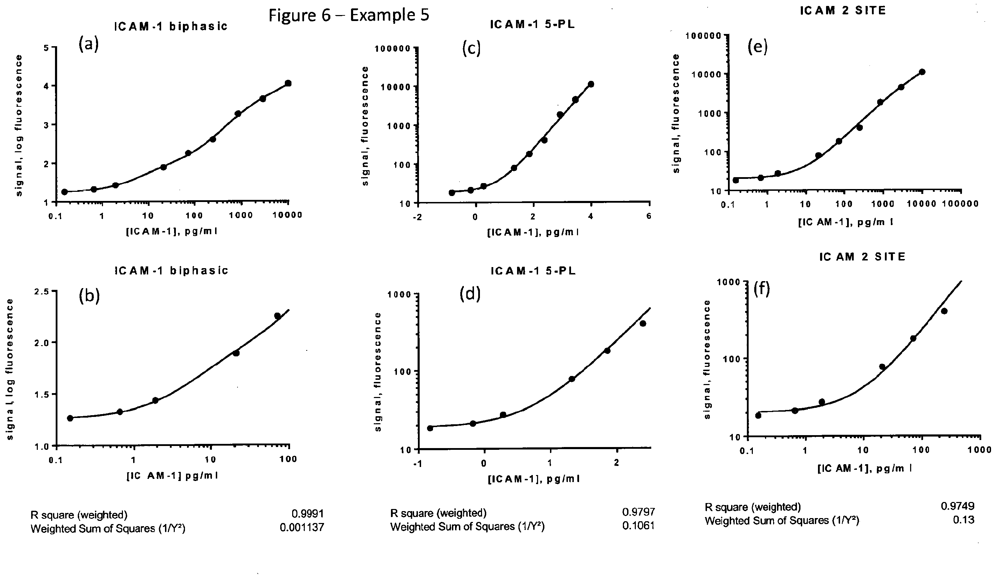

Figure 6 illustrates the standard curves of the analyte intercellular adhesion molecule 1 (ICAM-1);

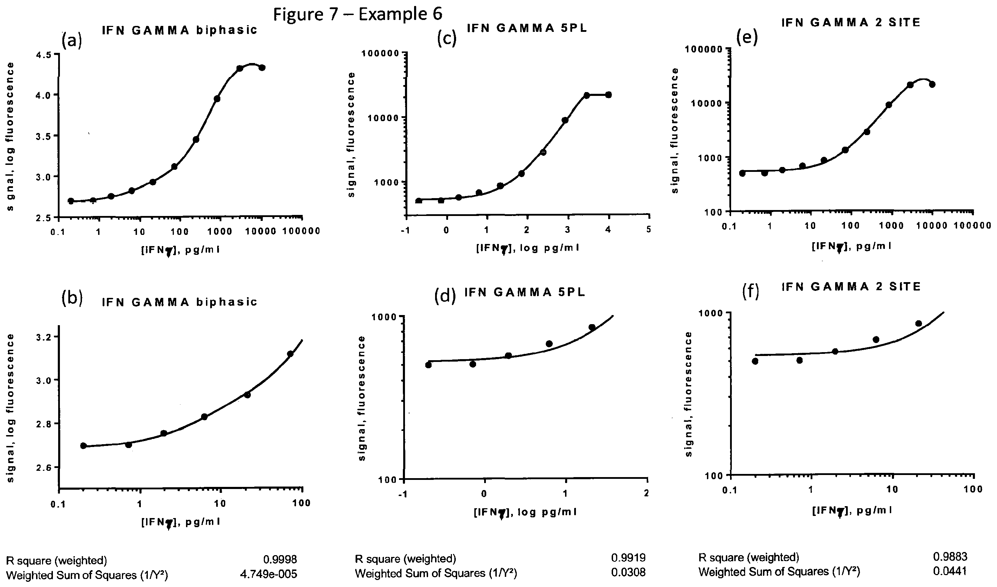

Figure 7 illustrates the standard curves of the analyte interferon gamma (IFN GAMMA);

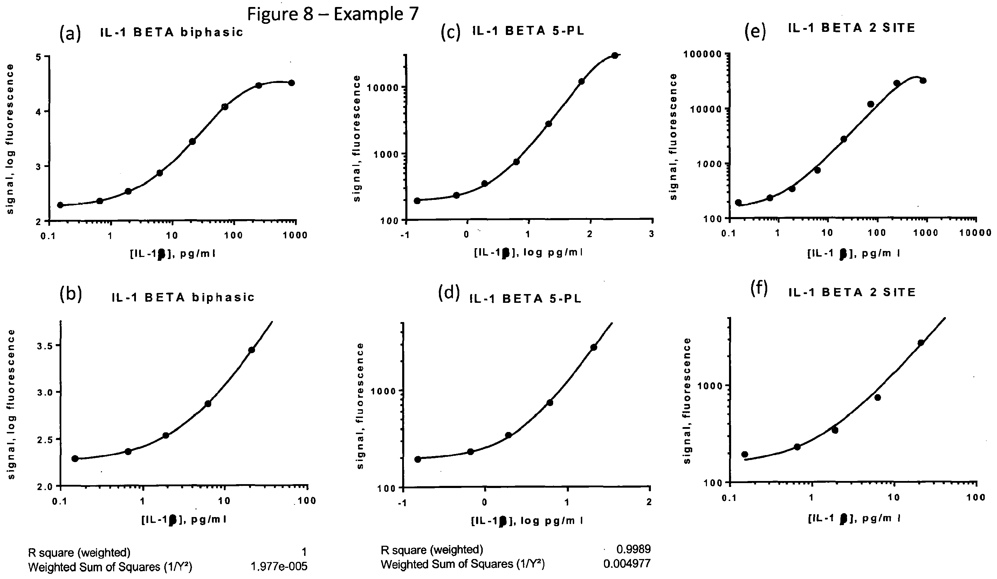

Figure 8 illustrates the standard curves of the analyte interleukin 1 beta (IL-1 BETA);

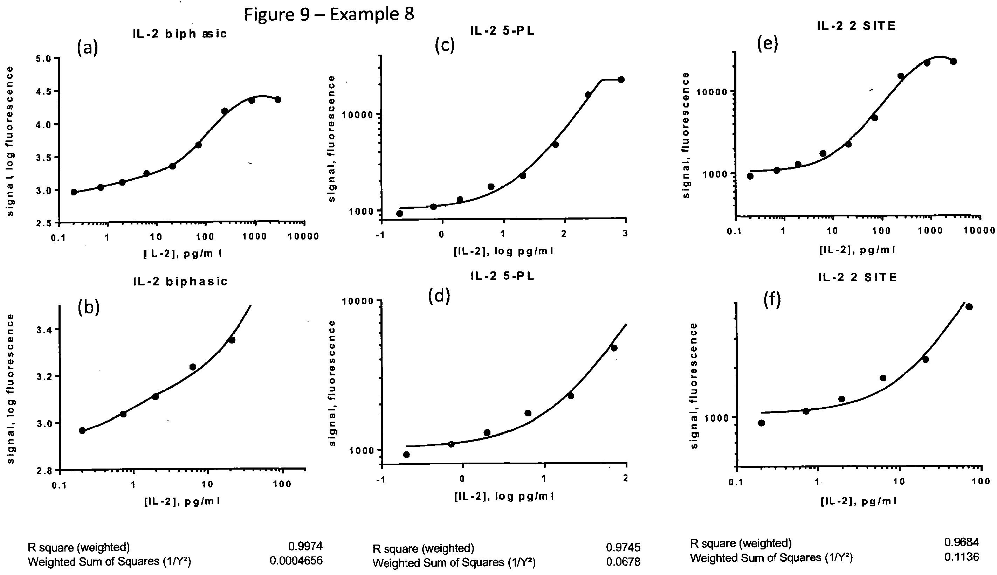

Figure 9 illustrates the standard curves of the analyte interleukin 2 (IL-2);

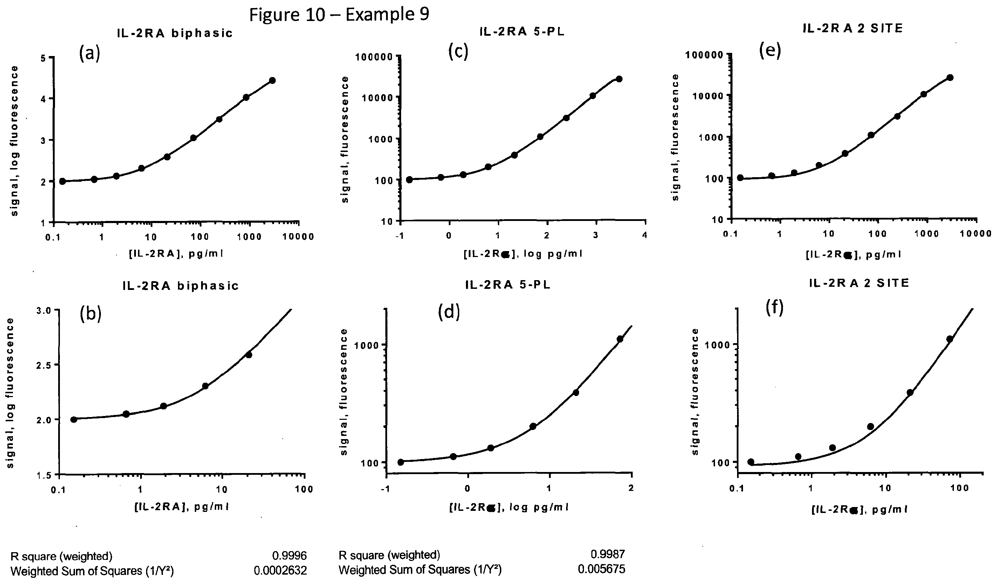

Figure 10 illustrates the standard curves of the analyte interleukin 2 receptor alpha (IL-2RA); and

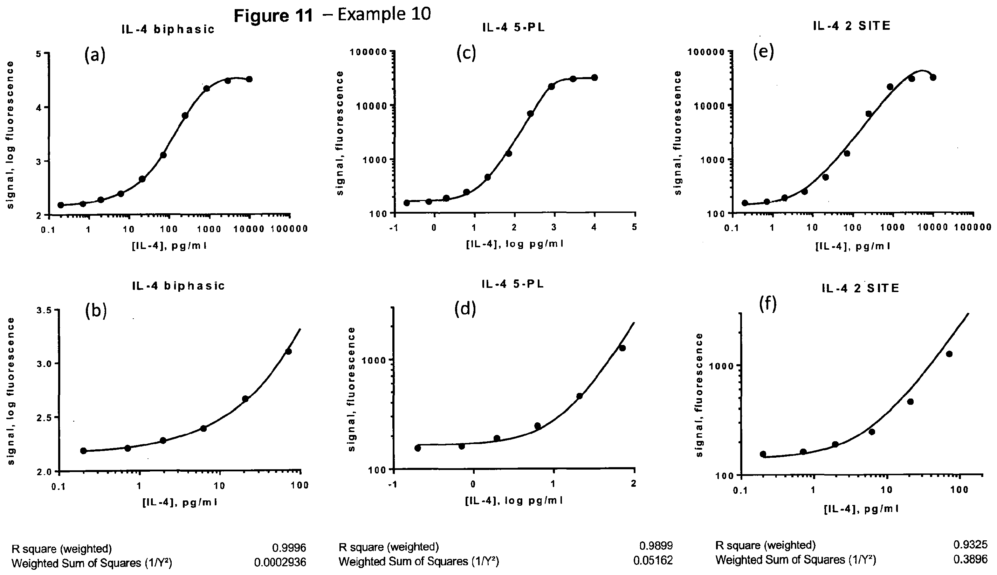

Figure 11 illustrates the standard curves of the analyte interleukin 4 (IL-4).

## Detailed Description

In general, the present invention is directed to a method of detecting the quantity of an analyte in a sample over a broad range of analyte concentrations, particularly at low analyte concentrations. Many analyte assaying techniques are known in the art, including many immunoassay techniques. The term 'assay' is used herein to mean a procedure for the detection or quantification of a soluble analyte. Immunoassay is an example of a type of assay suitable for use with the present invention. The term 'analyte' is used herein to mean any molecule or substance to be detected or quantified. The term 'sample' is used herein to mean an aliquot of any matter containing, or suspected to contain, an analyte of interest. Samples may include, for example, biological samples, or environmental samples or industrial samples. The term 'capture molecule' is used herein to mean a substrate-attached molecule that can be used to bind to the particular analyte being detected or quantified prior to the subsequent, or simultaneous binding of a detection molecule to the analyte attached to the capture molecule. The terms 'analyte-capture antibody complex' or 'analyte-capture molecule complex' is used herein to mean a complex comprising the capture antibody or molecule bound to the analyte being detected or quantified. The term 'detection molecule' is used herein to mean an antibody or molecule that can also be substrate-attached and binds to a particular analyte being detected or quantified and includes detection antibodies or molecules that can be coupled to signalling means that allow detection of the analyte bound to the capture antibody or molecule. Where the analyte is exposed to both capture and detection antibodies or molecules simultaneously, the detection antibody or molecule can bind to the analyte prior to, simultaneously with, or after the binding of analyte to the capture antibody or molecule and formation of the analyte-capture molecule complex. Therefore, the detection molecule or antibody can also form a complex with the analyte, termed analyte-detection molecule complex' or 'analyte-detection antibody complex', that can form prior to the binding of analyte to capture antibody or molecule.

Independent of the order in which capture and detection molecules or antibodies bind to the analyte, a complex is formed in which both capture and detection molecules or antibodies are bound to analyte, termed herein as 'an analyte-capture molecule-detection molecule-complex'.

The detection molecule or antibody could be secondary, i.e., as an example, first a detection molecule or antibody without a signalling moiety binds to the captured analyte and then a secondary detection molecule or antibody that carries (for example is conjugated to) a signalling moiety binds to the first detection antibody. Additionally, a detection molecule or antibody may be conjugated to a moiety that has affinity for and binds another signalling moiety-carrying molecule. A common example is the use of detection antibodies that carry biotin molecules. Biotin has very high affinity for avidin-like molecules, such as streptavidin and neutravidin. The avidin-like molecules in this case carry signalling moieties. Therefore, signalling is provided through a label that is either directly or indirectly bound to the detection antibody, or in the case of competitive assays can be directly attached to the non-analyte competing molecule. For example, the label may be selected from the group comprising colour-generating labels, chemiluminescent labels, fluorescent labels, phosphorescent labels, bioluminescent labels, electrochemiluminescent labels, crystalloluminescent labels, incandescent labels or radiation labels.

Signal may be detected using a variety of imaging devices, for example x-ray detectors, alpha, beta and gamma radiation detectors, CCD camera imaging devices, CMOS camera imaging devices, phosphorimagers, fluorimeters, flow cytometers, time resolved fluorescence spectrometers, fluorescence polarization analysers, quantitative polymerisation chain reaction reporters and spectrophotometers. The signal may be detected using X-ray film. Chemiluminescence may be detected using phosphorimagers. Ultraviolet (UV) and visible light (colours) may be detected through absorbance with a spectrophotometer or a UV/colour-sensitive CCD or CMOS camera.

Immunoassays, in general, involve incubation of solutions containing analyte with a capture antibody immobilised onto a substrate as a first step. In some cases of 'direct immunoassay', the analyte is immobilised onto a substrate for subsequent detection by an antibody. In systems that involve multiple washing steps, the analyte-containing solution is removed after an incubation period, which generally can be very few minutes to many hours and conducted at temperatures generally between 4°C and 37°C, but often at ambient temperature. Following the incubation with analyte-containing solution there can be a wash step to remove excess unattached analyte and analyte-containing solution followed by incubation with a solution containing a detection antibody. This incubation can be for the same length of time and temperatures as incubation of analyte solution with capture antibody. Following incubation with detection antibody there can be another wash step before either measurement of signal, if the detection antibody has a signalling label attached, or further incubation with a secondary detection antibody that has a signalling label attached, or another type of signalling system such as the avidin-based system wherein biotin is attached to the detection antibody and incubation is with a molecule related to avidin such as avidin itself, streptavidin or neutravidin that have a signalling label attached. Incubation in the avidin system is usually for a shorter period of time in comparison to antibodies because of the extremely high affinity of avidin-based molecules for biotin. In most cases a wash step is performed at the end of assay before signal detection.

In systems that avoid wash steps, incubation of the analyte containing solution can be with both capture and detection antibodies simultaneously. This is the case with the Alphalisa proximity assay from Perkin Elmer for example. In some systems, incubation of analyte-containing solution with both capture and detection antibody simultaneously is followed by incubation with a signal-generating solution without a prior wash step. An example of such a system is the Fireplex system from Abcam. Thus, many different and other configurations of assay design, some of which are described herein are used in soluble analyte quantification in assay systems and will be familiar to those experienced in the art and are included in the application of the invention described herein.

Standard curves obtained using either the 4-PL or 5-PL curve fitting models often do not give a good fit for data points at low concentrations. The adoption of these equations constrains the graph fitting to only apply a monophasic modelling of the data and therefore often inadequately fits curves through a series of points. The assumption made in application of both the 4-PL and 5-PL curve fitting models is that the relationship between analyte concentration and signal is monophasic, consistent with a single affinity state interaction between the analyte-binding molecules and analyte. This assumption of monophasic interaction is maintained in modelling of standard curves of many assay types. This assumption has also been maintained in immunoassay standard curve fitting in prior art, from the original conception of 4-PL and 5-PL use in immunoassay standard curve fitting to the current promotion of their use in immunoassay. This is demonstrated in recent reviewing of curve fitting in immunoassay and the current published description of curve fitting models incorporated in current commercial immunoassay kits (D. Davis, A. Zhang, C. Etienne and I. Huang. Principles of Curve Fitting for Multiplex Sandwich Immunoassays. Bio-Plex™ suspension array system tech note 2861; https://www.abcam.com/primary-antibodies/kd-value-a-quantitive-measurement-of-antibody-affinity). This is an understandable assumption because of the inability of the model for two separate molecule-ligand interaction events to dissect the immunoassay standard curves into two phases and thus generate biphasic modelling. This has also been demonstrated herein, where the model for two-site molecule-ligand interaction produces even worse curve fitting than both the 4-PL and 5-PL models (Figures 2-11). In the field of ligand-receptor binding there is an equation used to describe the concentration of bound ligand molecules $[L]_b$ when a protein molecule has two classes of non-interacting binding sites (FEBS Letters 392 (1996) 245-249):

$$[L]_b = \frac{[R_1] \times [L]}{K_2 + [L]} + \frac{[R_2] \times [L]}{K_2 + [L]}$$

where $[L]$ is the free ligand concentration, $K_1$, $[R_1]$, and $K_2$, $[R_2]$ are the dissociation constants and the binding capacity for binding site classes 1 and 2, respectively.

However, using this equation as a curve fitting model for immunoassay standard curve data does not give a good, or even practically useful fit to the data points (see Figures 2-11) and this modelling provides a significantly worse fit than the 5-PL modelling (Table 1).

Contrary to the established teaching in assay curve fitting and the indication, from the failure of 2-site modelling to provide functional curve fitting of immunoassay data, that immunoassay curve fitting should be conducted with monophasic modelling, the inventors have found that immunoassay standard curves can be better modelled by a combination of signal data transformation and biphasic curve fitting.

The inventors hypothesized that this two-site binding equation did not give a good fit to the data because the binding at low concentrations of analyte typically gives very low signal values. The inventors have found that by applying mathematical transformation functions to the signal results, to amplify the signal at the lower analyte concentrations of the curve relative to the signals derived from higher analyte concentrations, a novel biphasic curve model provides a curve fitting with highly statistically significant improvement in accuracy of analyte quantification in the lower concentration regions of soluble analyte immunoassays over the 5-PL model (see tables 1, 2, 3, 4 and 5). This improvement comprises (1) increased goodness of fit (increase in $r^2$ and decrease in sum of the squares (SSQ)), (2) sensitivity of the assay measured as a reduction of the lower limit of quantification (LLOQ) and (3) an increase in assay dynamic range.

The signal detected during binding of analyte to an antibody in immunoassay comprises three main components. First, is the signal derived from the specific interaction of analyte with antibody. Second, is the signal derived from nonspecific interaction of analyte with components in the assay system. Third, is background signal derived from the system in the absence of analyte. The methods for measurement of background (Bkd) and nonspecific binding will be well known to those skilled in the art of assay. As examples, in application of the combination of signal transformation and biphasic modelling described hereinbefore, the background signal, i.e. the signal generated in the absence of analyte can be provided by inclusion of points in the standard curve that are constructed for assay with zero concentration of analyte. The nonspecific binding could be quantified from generation of a standard curve in the absence of capture antibody, or the use of antibodies of the same isotype, but lacking significant affinity for the analyte.

In a preferred embodiment of the disclosure, the system incorporates the following equation 1, which models these three signal components:

## Equation 1

$$fY = \frac{fBmax1 \times [L]}{Kd1 + [L]} + \frac{fBmax2 \times [L]}{Kd2 + [L]} + f(NS \times [L]) + fBkd$$

Where $f$ is a mathematical transformation function amplifying the signal values at lower concentrations relative to the higher concentrations within the assay range including, but not exclusively, the following transformation factors:

$$\log_n x, \ \ln x, \ ASINH(x), and \ \sqrt[n]{x} \ or \ x^{\frac{1}{n}}$$

Where x=signal values and n is between 2 and 100. In a preferred embodiment n=10.

In a particularly preferred embodiment, $f = \log_{10} x$ and the logarithm to base 10 of the signal results is calculated.

Y is the signal generated at an analyte concentration [L];
Bmax1 is the maximal signal generated from a higher affinity analyte-capture molecule interaction;
Kd1 is the dissociation constant of the higher affinity analyte-capture molecule interaction;
Bmax2 is the maximal signal generated from a lower affinity analyte-capture molecule interaction; and
Kd2 is the dissociation constant of the lower affinity analyte-capture molecule interaction.
NS is the slope of change in signal derived from nonspecific binding of analyte with [L]
Bkd is the 'background' signal obtained when [L] = 0 (zero).

Where the background (Bkd) signal value is subtracted from the total signal data before transformation and standard curve generation, or its influence not modelled in the computation, then the fBkd term is removed from equation 1 providing equation 2:

## Equation 2

$$fY = \frac{fBmax1 \times [L]}{Kd1 + [L]} + \frac{fBmax2 \times [L]}{Kd2 + [L]} + f(NS \times [L])$$

Where the signal values derived from nonspecific binding of analyte (NS x [L]) are subtracted from the total signal data before transformation and standard curve generation, or its influence not modelled in the computation, then the f(NS x [L]) term is removed from equation 1 providing equation 3:

## Equation 3

$$fY = \frac{fBmax1 \times [L]}{Kd1 + [L]} + \frac{fBmax2 \times [L]}{Kd2 + [L]} + fBkd$$

Where both the signal values derived from nonspecific binding of analyte (NS x [L]) and background (Bkd) signal values are subtracted from the total signal data before transformation and standard curve generation, or their influence not modelled in the computation, then both the f(NS x [L]) and fBkd terms are removed from equation 1 providing equation 4:

## Equation 4

$$fY = \frac{fBmax1 \times [L]}{Kd1 + [L]} + \frac{fBmax2 \times [L]}{Kd2 + [L]}$$

The system described herein would incorporate options within the computation for choosing which of the four equations (1-4) to be applied to the curve fitting and equations 1-4 will be provided as options for curve fitting alongside the range of options usually provided for immunoassay curve fitting well known to those skilled in the art. The options described hereinbefore will be provided alongside a series of weighting options also well known to those skilled in the art of immunoassay curve fitting, including, but not exclusively, $1/y$, $1/y^2$, $1/x$, $1/x^2$ and $1/y^k$, where k is any number.

Examples

Figures 2 to 11 illustrate graphical representations of sets of data points collected from immunoassays quantifying ten different analytes. Each example was generated by incubation of a range of concentrations of analytes with Luminex Milliplex beads conjugated to capture antibodies for the analytes. Incubations were conducted at ambient temperature for 18hours in Aeirtec Incubation Buffer. Following washing to remove unbound analyte, the beads were incubated in Aeirtec Incubation Buffer with biotin-conjugated detection antibodies at 300 nanogram/ml with specificity for each analyte for 18 hours at ambient temperature. Following washing to remove unbound detection antibody, the beads were incubated in Aeirtec Incubation Buffer with R-phycoerythrin-conjugated streptavidin (streptavidin PE) at 0.5 microgram/ml for 1 hour at ambient temperature. Following washing to remove unbound streptavidin PE, the beads were analysed on a Luminex 200 analyser with xPONENT 3.1 software. Signal data were then transferred to a computer with programming for application of equation modelling to standard curve data. For each analyte, six graphs are illustrated in figures 2-11 and in each case the graphs are labelled 'a' to 'e': In each example, the graphs labelled 'a' and 'b' have used, as an example of the invention, the combination of log(10) transformation ($f = \log_{10} x$ where x is the signal results) with biphasic curve fitting model of the invention (equation 1) to fit the standard curve, the 'b' graph being an expanded view of the lower concentration range of data of the 'a' graph. In each example, the graphs labelled 'c' and 'd' have used the prior art 5-PL curve fitting model to fit the standard curve shown, the 'd' graph being an expanded view of the lower concentration range of data of the 'c' graph. And, in each example, the graphs labelled 'e' and 'f' have used the two-site binding model to fit the standard curve shown, the 'f' graph being an expanded view of the lower concentration range of data of the 'e' graph. Where no 'f' graph is shown, the standard curve did not fit the points at the lower concentration range.

Table 1 summarises the $R^2$ values for each example. $R^2$ is a statistical measure of how close the data points are to the fitted regression line. A value of 1.0 indicates that all the points are centred on the line, so the closer $R^2$ is to 1.0, the better the model fits the data. It is clear from a comparison of all the graphs, that the combined transformation and biphasic model of the invention provides the best fit.

Table 1 - Summary of $R^2$ values for the examples.

| Example | Analyte | $R^2$ Combined $f = \log_{10} x$ ** transformation and biphasic curve fitting model, $1/y^2$* | $R^2$ 5-PL curve fitting model, $1/y^2$* | $R^2$ Two-site curve fitting model, $1/y^2$* |
|---|---|---|---|---|
| 1 | CXCL16 | 0.999 | 0.9936 | 0.9902 |
| 2 | EOTAXIN 1 | 0.9999 | 0.9989 | 0.9648 |
| 3 | EOTAXIN 3 | 0.9998 | 0.9677 | n/a no fit seen |
| 4 | GM-CSF | 0.9969 | 0.9917 | n/a no fit seen |
| 5 | ICAM-1 | 0.9991 | 0.9797 | 0.9749 |
| 6 | IFN GAMMA | 0.9998 | 0.9919 | 0.9883 |
| 7 | IL-1 BETA | 1 | 0.9989 | n/a no fit seen |
| 8 | IL-2 | 0.9974 | 0.9745 | 0.9684 |
| 9 | IL-2RA | 0.9996 | 0.9987 | n/a no fit seen |

(continued)

| Example | Analyte | $R^2$ Combined $f = \log_{10} x$ ** transformation and biphasic curve fitting model, $1/y^{2*}$ | $R^2$ 5-PL curve fitting model, $1/y^{2*}$ | $R^2$ Two-site curve fitting model, $1/y^{2*}$ |
|---|---|---|---|---|
| 10 | IL-4 | 0.9996 | 0.9899 | 0.9325 |
| *, $1/y^2$ weighting was applied; **, transformation with f =Ln gave identical values to f =Log(10) | | | | |

The weighted sum of squares (SSQ $1/Y^2$) also gives an indication of the fit of the standard curves in each case. For a best fit, the weighted sum of squares should be minimised, i.e., the relative distances of the y values of the data from the y values of the curve should be minimised. A value of zero would indicate that all points are centred on the standard curve. Table 2 summarises the SSQ $1/Y^2$ -weighted values for each example.

Table 2 - Summary of SSQ $1/Y^{2*}$ weighted values for the examples.

| Example | Analyte | SSQ Combined $f = \log_{10} x$** transformation and biphasic curve fitting model, $1/y^{2*}$ | SSQ 5-PL curve fitting model, $1/y^{2*}$ | SSQ Two-site curve fitting model, $1/y^{2*}$ |
|---|---|---|---|---|
| 1 | CXCL16 | 0.0009054 | 0.04618 | 0.07012 |
| 2 | EOTAXIN 1 | 9.982e-005 | 0.006945 | 0.2333 |
| 3 | EOTAXIN 3 | 0.0001009 | 0.1349 | n/a no fit seen |
| 4 | GM-CSF | 3.186e-005 | 0.0359 | n/a no fit seen |
| 5 | ICAM-1 | 0.001137 | 0.1061 | 0.13 |
| 6 | IFN GAMMA | 4.749e-005 | 0.0308 | 0.0441 |
| 7 | IL-1 BETA | 1.977e-005 | 0.004977 | n/a no fit seen |
| 8 | IL-2 | 0.0004656 | 0.678 | 0.1136 |
| 9 | IL-2RA | 0.0002632 | 0.005675 | n/a no fit seen |
| 10 | IL-4 | 0.0002936 | 0.05162 | 0.3896 |
| *,$1/y^2$ weighting was applied; **,transformation with $f = \ln x$ gave identical values to $f = \log_{10} x$ | | | | |

Table 3 demonstrates the significant differences in $R^2$ and SSQ values derived from comparison of these indices between 5-PL modelling and examples of the combined transformation and biphasic modelling for the same 17 different analytes. Table 3 illustrates the results using four different transformation functions applied to the signal results ($x$), namely:

$f = \log_{10} x$; $f = \sqrt[10]{x}$ ; $f = x^{0.14562}$; and $f = ASINH(x)$.

.When the P value (provided in table 3) is less than 0.05 then there is, by convention, a statistically significant difference between the two values compared. In relation to the $R^2$ and SSQ values, the P values for the differences between the combined transformations with biphasic model and the 5-PL model are less than 0.005 to 0.002 in all cases, meaning that there is a negligible (<1 in 200 to <1 in 500) chance of the magnitude of differences between the curve fitting performance of these models occurring by random. Together, this tight consistency in improvement in fit provided with all transformation functions tested that amplify the signal values obtained at the lower analyte concentrations relative to the upper analyte concentration ranges demonstrates that whatever the transformation applied to the signal that achieves such signal amplification will result in a better curve fitting.

Table 3. Geometric means of $R^2$ and SSQ for the 5-PL modelling and examples of transformation with biphasic modelling

| | 5-PL $1/y^{2*}$ | $f = \log_{10} x$ transformation with biphasic $1/y^{2*}$ | P two-tailed | n |
|---|---|---|---|---|
| Geometric mean $R^2$ | 0.98267 | 0.99831 | <0.003 | 17 |

(continued)

| | 5-PL $1/y^{2*}$ | $f = \log_{10} x$ transformation with biphasic $1/y^{2*}$ | P two-tailed | n |
|---|---|---|---|---|
| Geometric mean SSQ | 0.02571 | 0.00050 | <0.002 | 17 |

| | 5-PL $1/y^{2*}$ | $f = \sqrt[10]{x}$ transformation with biphasic $1/y^{2*}$ | P two-tailed | n |
|---|---|---|---|---|
| Geometric mean $R^2$ | 0.98267 | 0.99801 | <0.005 | 17 |
| Geometric mean SSQ | 0.02571 | 0.00031 | <0.002 | 17 |

| | 5-PL $1/y^{2*}$ | $f = x^{0.14562}$ transformation with biphasic $1/y^{2*}$ | P two-tailed | n |
|---|---|---|---|---|
| Geometric mean $R^2$ | 0.98267 | 0.99778 | <0.003 | 17 |
| Geometric mean SSQ | 0.02571 | 0.00047 | <0.002 | 17 |
| | 5-PL $1/y^{2*}$ | $f = ASINH(x)$ transformation with biphasic $1/y^{2*}$ | P two-tailed | n |
| Geometric mean $R^2$ | 0.98267 | 0.99840 | <0.004 | 17 |
| Geometric mean SSQ | 0.02571 | 0.00041 | <0.002 | 17 |
| *, $1/y^2$ weighting was applied | | | | |

Therefore other methods for transformation for amplification of the signals obtained at the lower regions of analyte concentration to a greater extent than the upper regions, with the degree of amplification being inversely proportional to signal size, could be applied and will be known by those skilled in the art and familiar with data transformation.

The data in tables 1-3 were obtained from a sandwich ELISA assay in which the capture antibodies were attached to beads as substrate and detection antibodies were added into the assay free of substrate and in solution. The result of examination of standard curves generated in a sandwich ELISA format in which both capture and detection antibodies were attached to substrates at addition to the assay is shown in table 4. These assays were conducted using the Alphalisa proximity assay system according to the manufacturer's instructions. Again, this examination again demonstrated a highly statistically significant improvement in both R2 and SSQ values of curve fitting when using a combined signal transformation described hereinbefore with biphasic curve modelling. Overall, therefore the findings demonstrate that the invention provides significant benefit across a range of ELISA formats that at least includes different formats of sandwich ELISA and both proximity and non-proximity assays.

Table 4. Geometric means of $R^2$ and SSQ for the 5-PL, and transformation and biphasic modelling in Alphalisa proximity assay-generated standard curves

| | 5-PL $1/y^{2*}$ | $f = \log_{10} x$ transformation with biphasic $1/y^{2*}$ | P two-tailed | n |
|---|---|---|---|---|
| Geometric mean $R^2$ | 0.98267 | 0.99831 | <0.006 | 10 |
| Geometric mean SSQ | 0.04177 | 0.00080 | <0.006 | 10 |

The modelling using weighting can also be varied. Weighting using $1/y^2$ is one of the common default options in standard curve fitting. Other weighting options that are often default options and can be applied, but produced inferior curve fits than the $1/y^2$ weighting in the biphasic curve fitting, include the following: 1/y, 1/x and $1/x^2$. In some cases, the quality of curve fit using weighting $1/y^2$ is surpassed by weighting using $1/y^n$ with Y raised to the power of other values than 2, but this is preferably provided as an option of $1/y^n$ where n is between 1.5 and 10.

The significant improvement in curve fitting provided by the combined signal transformation with biphasic modelling indicates that the curves will more accurately quantify lower analyte concentrations and thereby provide greater assay sensitivity. The combined signal transformation with biphasic model and 5-PL model methods can be further compared in terms of effect on assay sensitivity by comparing the upper limit of quantification (ULOQ) for each method, the lower limit of quantification (LLOQ) and the dynamic range of quantification, which is the difference between the ULOQ and the LLOQ. Table 5 illustrates the geometric mean for each of these from results obtained from 17 different analytes.

Table 5 - Comparison of ULOQ, LLOQ and dynamic range between combined transformation with biphasic model and 5-PL model

| | $f = \log_{10} x$ transformation combined with biphasic model | 5-PL model | P(T<=t) two-tailed | Geometric mean of fold change |
|---|---|---|---|---|
| Geometric mean of ULOQ, pg/ml | 2400.776217 | 2096.3 | 0.28 | |
| Geometric mean of LLOQ, pg/ml | 0.31 | 2.75 | 0.000026 | 8.81 |
| Geometric mean of dynamic range, log units | 3.904 | 2.853 | 0.000028 | 9.7 |

As can be seen from Table 5, the transformation combined with biphasic model of the disclosure delivers a higher ULOQ, and a lower LLOQ than the 5-PL model, generating a greater dynamic range. In fact, the LLOQ is improved by a factor of almost 9, and the dynamic range by factor of almost 10. Again, when the P value (provided in table 5) is less than 0.05 then there is, by convention, a statistically significant difference between the two values compared. In relation to the LLOQ and dynamic range, the P values for the differences between the combined transformation with biphasic model and the 5-PL model are less than 0.00003, meaning that there is a negligible (<0.003% or <1 in 300,000) chance of the magnitude of differences between the analyte quantification performance of these models occurring by random. The highly statistically significant increase in assay sensitivity (decreased LLOQ) provided by the combined signal transformation and biphasic modelling, while ULOQ is maintained (P=0.28, no significant difference) generates the statistically significant increase in dynamic range, which also demonstrates a chance of occurring by random of <0.003% or <1 in 300,000.

The components for use in the method of the invention may be provided as a kit which includes the components required for conducting an assay and instructions for use of the components in conduct of the quantification of analyte concentrations within samples of unknown analyte concentration. Such a kit would include at least one assay substrate coated with an analyte capture molecule suitable for binding with the analyte. This could be in the form of a multi-well plate, such as a 96 well plate. Alternatively, the assay substrate could be in the form of a particle or bead coated with the analyte capture molecule. The kit would also include at least one detection molecule suitable for binding with the analyte. The kit would also include instructions for operation of a software to achieve the following in quantification of analyte concentrations within samples containing unknown concentrations of analyte.

The kit may include a concentrated sample of the analyte in question, known as a 'calibration analyte sample', to enable the user to conduct the assay at a series of known concentrations. Alternatively, the user may provide their own calibration analyte sample. Conducting the assay using at least six known concentrations of analyte is preferred. The values of detected analyte derived from the assay of known analyte concentrations in calibration standards is passed to a computer software and transformed through application of one of the optional transformations within the equations provided as part of the invention. The transformed values derived from detection of known concentration of analyte are then plotted in a graph ('calibration graph' or 'standard curve') against the known analyte concentration by the software to visualise the relationship between analyte detection values and the analyte concentration. The analyte concentrations are provided on a log value axis for convenience of illustration of the relationship. The software applies equations provided within the invention as options. An equation providing the best fit of points can be applied to define the relationship between the transformed analyte detection values and the known concentration of analyte. The curve fitting is then applied and a line of best fit drawn on the calibration graph.

When an equation within the invention has been selected for definition of the relationship between transformed analyte detection values and analyte concentration, the values of analyte concentration within the samples containing unknown analyte concentrations can be obtained through interpolation of the calibration graph by the software through application of the selected equation. The analyte detection values derived from the assay of samples of unknown analyte concentration, transformed in an identical fashion as the calibration standard values, are used to obtain values of analyte concentrations within the samples through interpolation using the selected equation.

Overall, the modelling of assay data provided in this invention generates significant improvement over the conventional methods in (1) curve fitting, (2) assay sensitivity and (3) dynamic range.

## Claims

1.   A method for determining the concentration of an analyte in a sample comprising the steps of:

    (a) providing a plurality of samples of the analyte at known concentrations;

(b) providing analyte capture molecules bound to a solid substrate, and detection molecules, the analyte capture molecules and detection molecules both suitable for binding with the analyte, wherein the detection molecules include signalling means to identify an interaction with the analyte;

(c) mixing the samples of analyte with the analyte capture molecules and detection molecules to form an analyte-capture molecule-detection molecule complex;

(d) detecting a signal resulting from the interaction of the analyte with the detection molecules;

(e) applying a mathematical transformation function to the detected signal;

(f) using a computer to mathematically model a biphasic standard curve of analyte concentration vs transformed detected signal of step (e) using the equation:

$$fY = \frac{fBmax1 \times [L]}{Kd1 + [L]} + \frac{fBmax2 \times [L]}{Kd2 + [L]}$$

where f is the mathematical transformation function used in step (e) and wherein the mathematical transformation function amplifies the signal values at lower concentrations relative to the higher concentrations within the assay range;

Y is the signal generated at an analyte concentration [L];

Bmax1 is the maximal signal generated from a higher affinity analyte-capture molecule interaction;

Kd1 is the dissociation constant of the higher affinity analyte-capture molecule interaction;

Bmax2 is the maximal signal generated from a lower affinity analyte-capture molecule interaction; and

Kd2 is the dissociation constant of the lower affinity analyte-capture molecule interaction.

(g) providing a sample of the analyte of unknown concentration;

(h) providing analyte capture molecules and detection molecules, the analyte capture molecules and detection molecules both suitable for binding with the analyte, wherein the detection molecules include signalling means to identify an interaction with the analyte ;

(i) mixing the samples of analyte with the analyte capture molecules and detection molecules to form an analyte-capture molecule-detection molecule complex;

(j) detecting a signal resulting from the interaction of the analyte with the detection molecules;

(k) applying the mathematical transformation function of step (e) to the detected signal of step (j); and

(l) determining the concentration of the analyte in the sample by correlating the transformed detected signal of step (k) with the analyte concentration using the biphasic standard curve generated in step (f).

2. A method for providing a calibration graph for an assay comprising the steps of:

(a) providing a plurality of samples of an analyte at known concentrations;

(b) providing analyte capture molecules bound to a solid substrate, and detection molecules, the analyte capture molecules and detection molecules both suitable for binding with the analyte, wherein the detection molecules include signalling means to identify an interaction with the analyte;

(c) mixing the samples of analyte with the analyte capture molecules and detection molecules to form an analyte-capture molecule-detection molecule complex;

(d) detecting a signal resulting from the interaction of the analyte with the detection molecules;

(e) applying a mathematical transformation function to the detected signal;

(f) using a computer to plot a graph of the known analyte concentration vs the transformed detected signal from step (e) and to mathematically model a biphasic standard curve of analyte concentration vs transformed detected signal of step (e) using the equation:

$$fY = \frac{fBmax1 \times [L]}{Kd1 + [L]} + \frac{fBmax2 \times [L]}{Kd2 + [L]}$$

where f is the mathematical transformation function used in step (e) and wherein the mathematical transformation function amplifies the signal values at lower concentrations relative to the higher concentrations within the assay range;

Y is the signal generated at an analyte concentration [L];

Bmax1 is the maximal signal generated from a higher affinity analyte-capture molecule interaction;
Kd1 is the dissociation constant of the higher affinity analyte-capture molecule interaction;
Bmax2 is the maximal signal generated from a lower affinity analyte-capture molecule interaction; and
Kd2 is the dissociation constant of the lower affinity analyte-capture molecule interaction.

3. A method according to Claim 1 or Claim 2, wherein the equation in step (f) further includes a term relating to a background signalling, Bkd, at analyte concentration of zero:

$$fY = \frac{fBmax1 \times [L]}{Kd1 + [L]} + \frac{fBmax2 \times [L]}{Kd2 + [L]} + fBkd$$

4. A method according to Claim 1 or Claim 2, wherein the equation in step (f) further includes a term relating to nonspecific signalling, NS, which varies with analyte concentration, f(NS x [L]):

$$fY = \frac{fBmax1 \times [L]}{Kd1 + [L]} + \frac{fBmax2 \times [L]}{Kd2 + [L]} f(NS \times [L])$$

5. A method according to Claim 1 or Claim 2, wherein the equation in step (f) further includes a term relating to background signalling, Bkd, at analyte concentration of zero, and a term relating to nonspecific signalling, NS, which varies with analyte concentration, f(NS x [L]):

$$fY = \frac{fBmax1 \times [L]}{Kd1 + [L]} + \frac{fBmax2 \times [L]}{Kd2 + [L]} + f(NS \times [L]) + fBkd$$

6. A method according to any preceding claim, wherein the transformation function, f, applied to the signal results in step (e) is selected from the group comprising: $\log_n x$, $\ln x$, $ASINH(x)$, $and$ $\sqrt[n]{x}$ $or$ $x^{\frac{1}{n}}$ where $x$ = signal results to be transformed, and $n$ is between 2 and 100.

7. A method for determining the concentration of an analyte in a sample according to claim 1 and any of claims 3 to 6 when dependent on claim 1, wherein the number of samples of known concentration in step (a) is at least six.

8. A method for determining the concentration of an analyte in a sample according to claim 1 and any of claims 3 to 6 when dependent on claim 1, wherein the signalling means of the detection molecule is selected from the group comprising colour-generating labels, chemiluminescent labels, fluorescent labels, phosphorescent labels, bioluminescent labels, electrochemiluminescent labels, crystalloluminescent labels, incandescent labels or radiation labels.

9. A method for determining the concentration of an analyte in a sample according to claim 1 and any of claims 3 to 6 when dependent on claim 1, wherein in steps (d) and (j) the signal is detected using a device selected from the group comprising x-ray detectors, alpha, beta and gamma radiation detectors, CCD camera imaging devices, CMOS camera imaging devices, phosphorimagers, fluorimeters, flow cytometers, time resolved fluorescence spectrometers, fluorescence polarization analysers, quantitative polymerization chain reaction reporters and spectrophotometers.

10. A method for determining the concentration of an analyte in a sample according to claim 1 and any of claims 3 to 6 when dependent on claim 1, wherein the assay is an immunoassay.

11. A computer programmed with computer software for determining the concentration of an analyte in a sample using the method of claim 1 and the method of any of claims 3 to 10, wherein the computer software_is configured to:

(i) receive the signal detected in step (d) of the method of claim 1 and apply a mathematical transformation function to the detected signal, or receive the transformed detected signal of step (e) of the method of claim 1;
(ii) plot a biphasic standard curve of analyte concentration vs transformed detected signal using the equation according to step (f) of the method of claim 1;
(iii) receive the signal detected in step (j) of the method of claim 1 from an analyte of unknown concentration or receive the transformed detected signal of step (k) of the method of claim 1; and
(iv) determine the concentration of the analyte of unknown concentration using the biphasic standard curve.

## Patentansprüche

1. Verfahren zum Bestimmen der Konzentration eines Analyten in einer Probe, das die folgenden Schritte beinhaltet:

(a) Bereitstellen mehrerer Proben des Analyten in bekannten Konzentrationen;
(b) Bereitstellen von an ein festes Substrat gebundenen Analyteinfangmolekülen und Detektionsmolekülen, wobei sowohl die Analyteinfangmoleküle als auch die Detektionsmoleküle zur Bindung an den Analyt geeignet sind, wobei die Detektionsmoleküle Signalisierungsmittel zum Identifizieren einer Interaktion mit dem Analyt umfassen;
(c) Mischen der Analytproben mit den Analyteinfangmolekülen und den Detektionsmolekülen, um einen Analyteinfangmolekül-Detektionsmolekül-Komplex zu bilden;
(d) Detektieren eines Signals, das aus der Interaktion des Analyten mit den Detektionsmolekülen resultiert;
(e) Anwenden einer mathematischen Transformationsfunktion auf das detektierte Signal;
(f) mathematisches Modellieren, mittels eines Computers, einer biphasischen Standardkurve von Analytkonzentration gegenüber transformiertem detektiertem Signal aus Schritt (e) anhand der folgenden Gleichung:

$$fY = \frac{fBmax1 \times [L]}{Kd1 + [L]} + \frac{fBmax2 \times [L]}{Kd2 + [L]}$$

wobei $f$ die in Schritt (e) verwendete mathematische Transformationsfunktion ist und wobei die mathematische Transformationsfunktion die Signalwerte bei niedrigeren Konzentrationen im Vergleich zu den höheren Konzentrationen innerhalb des Assaybereichs verstärkt;
Y das bei einer Analytkonzentration [L] erzeugte Signal ist;
Bmax1 das maximale Signal ist, das aus einer Interaktion des Analyteinfangmoleküls mit höherer Affinität erzeugt wird;
Kd1 die Dissoziationskonstante der Interaktion des Analyteinfangmoleküls mit höherer Affinität ist;
Bmax2 das maximale Signal ist, das aus einer Interaktion des Analyteinfangmoleküls mit niedrigerer Affinität erzeugt wird; und
Kd2 die Dissoziationskonstante der Interaktion des Analyteinfangmoleküls mit niedrigerer Affinität ist;

(g) Bereitstellen einer Probe des Analyten mit unbekannter Konzentration;
(h) Bereitstellen von Analyteinfangmolekülen und Detektionsmolekülen, wobei sowohl die Analyteinfangmoleküle als auch die Detektionsmoleküle zur Bindung mit dem Analyt geeignet sind, wobei die Detektionsmoleküle Signalisierungmittel zum Identifizieren einer Interakation mit dem Analyt umfassen;
(i) Mischen der Analytproben mit den Analyteinfangmolekülen und Detektionsmolekülen, um einen Analyteinfangmolekül-Detektionsmolekül-Komplex zu bilden;
(j) Detektieren eines Signals, das aus der Interaktion des Analyten mit den Detektionsmolekülen resultiert;
(k) Anwenden der mathematischen Transformationsfunktion aus Schritt (e) auf das detektierte Signal aus Schritt (j); und
(l) Bestimmen der Konzentration des Analyten in der Probe durch Korrelieren des transformierten detektierten Signals aus Schritt (k) mit der Analytkonzentration anhand der in Schritt (f) erzeugten biphasischen Standardkurve.

2. Verfahren zum Bereitstellen einer Kalibrierungskurve für einen Assay, das die folgenden Schritte beinhaltet:

(a) Bereitstellen mehrerer Proben eines Analyten in bekannten Konzentrationen;
(b) Bereitstellen von an ein festes Substrat gebundenen Analyteinfangmolekülen und Detektionsmolekülen, wobei sowohl die Analyteinfangmoleküle als auch die Detektionsmoleküle zur Bindung an den Analyt geeignet sind, wobei die Detektionsmoleküle Signalisierungsmittel zum Identifizieren einer Interaktion mit dem Analyt umfassen;

(c) Mischen der Analytproben mit den Analyteinfangmolekülen und den Detektionsmolekülen, um einen Analyteinfangmolekül-Detektionsmolekül-Komplex zu bilden;

(d) Detektieren eines Signals, das aus der Interaktion des Analyten mit den Detektionsmolekülen resultiert;

(e) Anwenden einer mathematischen Transformationsfunktion auf das detektierte Signal;

(f) Benutzen eines Computers zum Plotten einer Kurve der bekannten Analytkonzentration gegenüber transformiertem detektiertem Signal aus Schritt (3) und zum mathematischen Modellieren einer biphasischen Standardkurve von Analytkonzentration gegenüber transformiertem detektiertem Signal aus Schritt (e) anhand der folgenden Gleichung:

$$fY = \frac{fBmax1 \times [L]}{Kd1 + [L]} + \frac{fBmax2 \times [L]}{Kd2 + [L]}$$

wobei $f$ die in Schritt (e) verwendete mathematische Transformationsfunktion ist und wobei die mathematische Transformationsfunktion die Signalwerte bei niedrigeren Konzentrationen im Vergleich zu den höheren Konzentrationen innerhalb des Assaybereichs verstärkt;

Y das bei einer Analytkonzentration [L] erzeugte Signal ist;

Bmax1 das maximale Signal ist, das aus einer Interaktion des Analyteinfangmoleküls mit höherer Affinität erzeugt wird;

Kd1 die Dissoziationskonstante der Interaktion des Analyteinfangmoleküls mit höherer Affinität ist;

Bmax2 das maximale Signal ist, das aus einer Interaktion des Analyteinfangmoleküls mit niedrigerer Affinität erzeugt wird; und

Kd2 die Dissoziationskonstante der Interaktion des Analyteinfangmoleküls mit niedrigerer Affinität ist.

3. Verfahren nach Anspruch 1 oder Anspruch 2, wobei die Gleichung in Schritt (f) ferner einen Term enthält, der sich auf die Hintergrundsignalisierung, Bkd, bei einer Analytkonzentration von Null bezieht:

$$fY = \frac{fBmax1 \times [L]}{Kd1 + [L]} + \frac{fBmax2 \times [L]}{Kd2 + [L]} + fBkd \quad .$$

4. Verfahren nach Anspruch 1 oder Anspruch 2, wobei die Gleichung in Schritt (f) ferner einen Term enthält, der sich auf unspezifische Signalgebung, NS, bezieht, der mit der Analytkonzentration variiert, $f(NS \times [L])$:

$$fY = \frac{fBmax1 \times [L]}{Kd1 + [L]} + \frac{fBmax2 \times [L]}{Kd2 + [L]} f(NS \times [L]) \quad .$$

5. Verfahren nach Anspruch 1 oder Anspruch 2, wobei die Gleichung in Schritt (f) ferner einen Term umfasst, der sich auf die Hintergrundsignalisierung, Bkd, bei einer Analytkonzentration von Null bezieht, und einen Term, der sich auf die unspezifische Signalisierung, NS, bezieht, die mit der Analytkonzentration variiert, $f(NS \times [L])$:

$$fY = \frac{fBmax1 \times [L]}{Kd1 + [L]} + \frac{fBmax2 \times [L]}{Kd2 + [L]} + f(NS \times [L]) + fBkd \quad .$$

6. Verfahren nach einem vorherigen Anspruch, wobei die Transformationsfunktion $f$, die in Schritt (e) auf das Signal angewendet wird, aus der Gruppe ausgewählt wird, die Folgendes umfasst: $\log_n x$, $\ln x$, $ASINH(x)$ und $\sqrt[n]{x}$ oder $x^{\frac{1}{n}}$, wobei x = zu transformierende Signalergebnisse ist und n zwischen 2 und 100 liegt.

7. Verfahren zum Bestimmen der Konzentration eines Analyten in einer Probe nach Anspruch 1 und einem der Ansprüche 3 bis 6 wenn abhängig von Anspruch 1, wobei die Anzahl von Proben mit bekannter Konzentration in Schritt (a) mindestens sechs beträgt.

8. Verfahren zum Bestimmen der Konzentration eines Analyten in einer Probe nach Anspruch 1 und einem der Ansprüche 3 bis 6 wenn abhängig von Anspruch 1, wobei die Signalisierungsmittel des Detektionsmoleküls aus der Gruppe ausgewählt sind, die farbgebende Markierungen, chemilumineszente Markierungen, fluoreszierende Markierungen, phosphoreszierende Markierungen, biolumineszente Markierungen, elektrochemilumineszente Markierungen, kristallolumineszente Markierungen, inkandeszente Markierungen oder Strahlungsmarkierungen umfasst.

**9.** Verfahren zum Bestimmen der Konzentration eines Analyten in einer Probe nach Anspruch 1 und einem der Ansprüche 3 bis 6 wenn abhängig von Anspruch 1, wobei in den Schritten (d) und (j) das Signal mit Hilfe einer Vorrichtung detektiert wird, die aus der Gruppe ausgewählt ist, die Röntgendetektoren, Alpha-, Beta- und Gamma-Strahlungsdetektoren, CCD-Kamera-Bildgebungsvorrichtungen, CMOS-Kamera-Bildgebungsvorrichtungen, Phosphorimager, Fluorimeter, Durchflusszytometer, zeitaufgelöste Fluoreszenzspektrometer, Fluoreszenzpolarisations-Analysatoren, quantitative Polymerisationskettenreaktions-Reporter und Spektralphotometer ausgewählt ist.

**10.** Verfahren zum Bestimmen der Konzentration eines Analyten in einer Probe nach Anspruch 1 und einem der Ansprüche 3 bis 6 wenn abhängig von Anspruch 1, wobei der Assay ein Immunoassay ist.

**11.** Computer, der mit Computersoftware zum Bestimmen der Konzentration eines Analyten in einer Probe mit dem Verfahren nach Anspruch 1 und einem der Ansprüche 3 bis 10 programmiert ist, wobei die Computersoftware konfiguriert ist zum:

(i) Empfangen des in Schritt (d) des Verfahrens nach Anspruch 1 detektierten Signals und Anwenden einer mathematischen Transformationsfunktion auf das detektierte Signal oder Empfangen des transformierten detektierten Signals aus Schritt (e) des Verfahrens nach Anspruch 1;
(ii) Plotten einer biphasischen Standardkurve von Analytkonzentration gegenüber transformiertem detektiertem Signal anhand der Gleichung gemäß Schritt (f) des Verfahrens nach Anspruch 1;
(iii) Empfangen des in Schritt (j) des Verfahrens nach Anspruch 1 erkannten Signals von einem Analyten unbekannter Konzentration oder Empfangen des transformierten erkannten Signals aus Schritt (k) des Verfahrens nach Anspruch 1; und
(iv) Bestimmen der Konzentration des Analyten unbekannter Konzentration anhand der biphasischen Standardkurve.

**Revendications**

**1.** Procédé de détermination de la concentration d'un analyte dans un échantillon comprenant les étapes consistant à :

(a) fournir une pluralité d'échantillons de l'analyte à des concentrations connues,
(b) fournir des molécules de capture d'analyte liées à un substrat solide, et des molécules de détection, les molécules de capture d'analyte et les molécules de détection convenant toutes les deux pour se lier à l'analyte, dans lequel les molécules de détection comprennent des moyens de signalisation pour identifier une interaction avec l'analyte ;
(c) mélanger les échantillons d'analyte avec les molécules de capture d'analyte et les molécules de détection afin de former un complexe de molécules de capture d'analyte et de molécules de détection ;
(d) détecter un signal résultant de l'interaction de l'analyte avec les molécules de détection ;
(e) appliquer une fonction de transformation mathématique au signal détecté ;
(f) utiliser un ordinateur pour modéliser une courbe standard biphasique de concentration d'analyte par rapport au signal détecté transformé de l'étape (e) en utilisant l'équation :

$$fY = \frac{fBmax1 \times [L]}{Kd1 + [L]} + \frac{fBmax2 \times [L]}{Kd2 + [L]}$$

dans laquelle f est la fonction de transformation mathématique utilisée à l'étape (e) et dans laquelle la fonction de transformation mathématique amplifie les valeurs du signal à des concentrations plus basses par rapport aux concentrations plus hautes dans la plage de l'essai ;
Y est le signal généré à une concentration d'analyte [L] ;
Bmax1 est le signal maximal généré d'une interaction de molécules de capture d'analyte de plus haute affinité ;

Kd1 est la constante de dissociation de l'interaction de molécules de capture d'analyte de plus haute affinité ;
Bmax2 est le signal maximal généré d'une interaction de molécules de capture d'analyte de plus faible affinité ; et
Kd2 est la constante de dissociation de l'interaction de molécules de capture d'analyte de plus faible affinité.

(g) fournir un échantillon de l'analyte de concentration inconnue ;
(h) fournir des molécules de capture d'analyte et des molécules de détection, les molécules de capture d'analyte et les molécules de détection convenant toutes les deux pour se lier à l'analyte, dans lequel les molécules de détection comprennent des moyens de signalisation pour identifier une interaction avec l'analyte ;
(i) mélanger les échantillons d'analyte avec les molécules de capture d'analyte et les molécules de détection pour former un complexe de molécules de capture d'analyte et de molécules de détection ;
(j) détecter un signal résultant de l'interaction de l'analyte avec les molécules de détection ;
(k) appliquer la fonction de transformation mathématique de l'étape (e) au signal détecté de l'étape (j) ; et
(l) déterminer la concentration de l'analyte dans l'échantillon en mettant en corrélation le signal détecté transformé de l'étape (k) avec la concentration d'analyte en utilisant une courbe standard biphasique générée à l'étape (f).

2. Procédé destiné à fournir un graphique d'étalonnage pour un essai comprenant les étapes consistant à :

(a) fournir une pluralité d'échantillons d'un analyte à des concentrations connues,
(b) fournir des molécules de capture d'analyte liées à un substrat solide, et des molécules de détection, les molécules de capture d'analyte et les molécules de détection convenant toutes les deux pour se lier à l'analyte, dans lequel les molécules de détection comprennent des moyens de signalisation pour identifier une interaction avec l'analyte ;
(c) mélanger les échantillons d'analyte avec les molécules de capture d'analyte et les molécules de détection afin de former un complexe de molécules de capture d'analyte et de molécules de détection ;
(d) détecter un signal résultant de l'interaction de l'analyte avec les molécules de détection ;
(e) appliquer une fonction de transformation mathématique au signal détecté ;
(f) utiliser un ordinateur pour tracer un graphique de la concentration d'analyte connue par rapport au signal détecté transformé de l'étape (e) et pour modéliser mathématiquement une courbe standard biphasique de concentration d'analyte par rapport au signal détecté transformé de l'étape (e) en utilisant l'équation :

$$fY = \frac{fBmax1 \times [L]}{Kd1 + [L]} + \frac{fBmax2 \times [L]}{Kd2 + [L]}$$

dans laquelle f est la fonction de transformation mathématique utilisée à l'étape (e) et dans laquelle la fonction de transformation mathématique amplifie les valeurs du signal à des concentrations plus basses par rapport aux concentrations plus hautes dans la plage de l'essai ;
Y est le signal généré à une concentration d'analyte [L] ;
Bmax1 est le signal maximal généré d'une interaction de molécules de capture d'analyte de plus haute affinité ;
Kd1 est la constante de dissociation de l'interaction de molécules de capture d'analyte de plus haute affinité ;
Bmax2 est le signal maximal généré d'une interaction de molécules de capture d'analyte de plus faible affinité ; et
Kd2 est la constante de dissociation de l'interaction de molécules de capture d'analyte de plus faible affinité.

3. Procédé selon la revendication 1 ou la revendication 2, dans lequel l'équation à l'étape (f) comprend en outre un terme associé à une signalisation de fond, Bkd, à la concentration d'analyte de zéro :

$$fY = \frac{fBmax1 \times [L]}{Kd1 + [L]} + \frac{fBmax2 \times [L]}{Kd2 + [L]} + fBkd$$

4. Procédé selon la revendication 1 ou la revendication 2, dans lequel l'équation à l'étape (f) comprend en outre un terme associé à une signalisation non spécifique, NS, qui varie avec la concentration d'analyte, f(NS x [L]) :

$$fY = \frac{fBmax1 \times [L]}{Kd1 + [L]} + \frac{fBmax2 \times [L]}{Kd2 + [L]} f(NS \times [L])$$

5. Procédé selon la revendication 1 ou la revendication 2, dans lequel l'équation à l'étape (f) comprend en outre un terme associé à une signalisation de fond, Bkd, à la concentration d'analyte de zéro, et un terme associé à une signalisation non spécifique, NS, qui varie avec la concentration d'analyte, f(NS x [L]) :

$$fY = \frac{fBmax1 \times [L]}{Kd1 + [L]} + \frac{fBmax2 \times [L]}{Kd2 + [L]} + f(NS \times [L]) + fBkd$$

6. Procédé selon l'une quelconque des revendications précédentes, dans lequel la fonction de transformation, f, appliquée aux résultats du signal à l'étape (e) est

$$\sqrt[n]{x}$$

$$x^{\frac{1}{n}}$$

sélectionnée parmi le groupe comprenant : $Log_n x$, 1nx, *ASINH(x)*, *et*
*ou*
où *x* = les résultats du signal à transformer, et *n* est d'entre 2 et 100.

7. Procédé de détermination de la concentration d'un analyte dans un échantillon selon la revendication 1 et selon l'une quelconque des revendications 3 à 6 lorsque dépendantes de la revendication 1, dans lequel le nombre d'échantillons de concentration connue à l'étape (a) est d'au moins six.

8. Procédé de détermination de la concentration d'un analyte dans un échantillon selon la revendication 1 et selon l'une quelconque des revendications 3 à 6 lorsque dépendantes de la revendication 1, dans lequel les moyens de signalisation des molécules de détection sont sélectionnés parmi le groupe comprenant des marqueurs produisant de la couleur, des marqueurs chimioluminescents, des marqueurs fluorescents, des marqueurs phosphorescents, des marqueurs bioluminescents, des marqueurs électrochimioluminescents, des marqueurs cristalloluminescents, des marqueurs incandescents ou des marqueurs de radiation.

9. Procédé de détermination de la concentration d'un analyte dans un échantillon selon la revendication 1 et selon l'une quelconque des revendications 3 à 6 lorsque dépendantes de la revendication 1, dans lequel aux étapes (d) et (j) le signal est détecté en utilisant un appareil sélectionné parmi le groupe comprenant des détecteurs de rayons X, des détecteurs de radiation alpha, bêta et gamma, des appareils d'imagerie à caméra CCD, des appareils d'imageries à caméra CMOS, des phosphorimètres, des fluorimètres, des cytomètres en flux, des spectromètres de fluorescence résolus dans le temps, des analyseurs de polarisation de fluorescence, des rapporteurs de réaction de polymérisation en chaîne quantitative et des spectrophotomètres.

10. Procédé de détermination de la concentration d'un analyte dans un échantillon selon la revendication 1 et l'une quelconque des revendications 3 à 6 lorsque dépendantes de la revendication 1, dans lequel l'essai est un immunoessai,

11. Ordinateur programmé avec du logiciel informatique pour déterminer la concentration d'un analyte dans un échantillon en utilisant le procédé selon la revendication 1 et le procédé selon l'une quelconque des revendications 3 à 10, dans lequel le logiciel informatique est configuré pour :

(i) recevoir le signal détecté à l'étape (d) du procédé selon la revendication 1 et appliquer une fonction de transformation mathématique au signal détecté, ou recevoir le signal détecté transformé de l'étape (e) du procédé selon la revendication 1

(ii) tracer une courbe standard biphasique de concentration d'analyte par rapport au signal détecté transformé en utilisant l'équation conformément à l'étape (f) du procédé selon la revendication 1 ;

(iii) recevoir le signal détecté à l'étape (j) du procédé selon la revendication 1 d'un analyte de concentration inconnue ou recevoir le signal détecté transformé de l'étape (k) du procédé selon la revendication 1 ; et

(iv) déterminer la concentration de l'analyte de concentration inconnue en utilisant la courbe standard biphasique.

Figure 1

analyte + capture antibody → + detection antibody → + signal

Figure 2 – Example 1

24

# Figure 3 - Example 2

EOTAXIN 1 biphasic — (a)

EOTAXIN 1 5-PL — (c)

EOTAXIN 1 2 SITE — (e)

## Figure 3 – Example 2

EOTAXIN 1 biphasic — (b)

EOTAXIN 1 5-PL — (d)

EOTAXIN 1 2 SITE — (f)

| | |
|---|---|
| R square (weighted) | 0.9999 |
| Weighted Sum of Squares (1/Y²) | 9.982e-005 |

| | |
|---|---|
| R square (weighted) | 0.9989 |
| Weighted Sum of Squares (1/Y²) | 0.006945 |

| | |
|---|---|
| R square (weighted) | 0.9648 |
| Weighted Sum of Squares (1/Y²) | 0.2333 |

EP 3 894 855 B1

Figure 4 – Example 3

# Figure 5 – Example 4

(a) **GM-CSF biphasic**

(b) **GM-CSF biphasic**

(c) **GM-CSF 5PL**

(d) **GM-CSF 5PL**

(e) **GM-CSF 2 SITE**

| | |
|---|---|
| R square (weighted) | 0.9969 |
| Weighted Sum of Squares (1/Y^5) | 3.186e-005 |

| | |
|---|---|
| R square (weighted) | 0.9917 |
| Weighted Sum of Squares (1/Y²) | 0.0359 |

EP 3 894 855 B1

# Figure 6 – Example 5

(a) ICAM-1 biphasic

(b) ICAM-1 biphasic

(c) ICAM-1 5-PL

(d) ICAM-1 5-PL

(e) ICAM 2 SITE

(f) ICAM 2 SITE

| | |
|---|---|
| R square (weighted) | 0.9991 |
| Weighted Sum of Squares (1/Y²) | 0.001137 |

| | |
|---|---|
| R square (weighted) | 0.9797 |
| Weighted Sum of Squares (1/Y²) | 0.1061 |

| | |
|---|---|
| R square (weighted) | 0.9749 |
| Weighted Sum of Squares (1/Y²) | 0.13 |

EP 3 894 855 B1

Figure 7 – Example 6

(a) IFN GAMMA biphasic

(b) IFN GAMMA biphasic

(c) IFN GAMMA 5PL

(d) IFN GAMMA 5PL

(e) IFN GAMMA 2 SITE

(f) IFN GAMMA 2 SITE

| R square (weighted) | 0.9998 |
| Weighted Sum of Squares (1/Y²) | 4.749e-005 |

| R square (weighted) | 0.9919 |
| Weighted Sum of Squares (1/Y²) | 0.0308 |

| R square (weighted) | 0.9883 |
| Weighted Sum of Squares (1/Y²) | 0.0441 |

EP 3 894 855 B1

EP 3 894 855 B1

Figure 8 – Example 7

(a) IL-1 BETA biphasic
(b) IL-1 BETA biphasic
(c) IL-1 BETA 5-PL
(d) IL-1 BETA 5-PL
(e) IL-1 BETA 2 SITE
(f) IL-1 BETA 2 SITE

Figure 9 – Example 8

EP 3 894 855 B1

EP 3 894 855 B1

## Figure 10 – Example 9

| R square (weighted) | 0.9996 | R square (weighted) | 0.9987 |
|---|---|---|---|
| Weighted Sum of Squares (1/Y²) | 0.0002632 | Weighted Sum of Squares (1/Y²) | 0.005675 |

EP 3 894 855 B1

## Figure 11 — Example 10

**IL-4 biphasic** (a)

**IL-4 5-PL** (c)

**IL-4 2 SITE** (e)

**IL-4 biphasic** (b)

**IL-4 5-PL** (d)

**IL-4 2 SITE** (f)

| R square (weighted) | 0.9996 |
| --- | --- |
| Weighted Sum of Squares (1/Y²) | 0.0002936 |

| R square (weighted) | 0.9899 |
| --- | --- |
| Weighted Sum of Squares (1/Y²) | 0.05162 |

| R square (weighted) | 0.9325 |
| --- | --- |
| Weighted Sum of Squares (1/Y²) | 0.3896 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014256588 A
- WO 2017032848 A
- WO 2012087840 A

**Non-patent literature cited in the description**

- **YALOW RS** ; **BERSON SA**. Assay of plasma insulin in human subjects by immunological methods. *Nature*, 1959, vol. 184, 1648-1649
- **VAN WEEMEN BK** ; **SCHUURS AHWM**. Immunoassay using antigen-enzyme conjugates.. *FEBS Lett.*, 1971, vol. 15 (3), 232-236
- **ENGVALL E** ; **PERLMANN P.** Enzyme-linked immunosorbent assay (ELISA): quantitative assay of immunoglobulin G.. *Immunochemistry*, 1971, vol. 8 (9), 871-874
- Determination of antibody affinity and kinetic binding constatis in Gyrolab Bioaffy microfluidic CD. **MIKAEL KARLSSON**. Master Thesis. Linköping University, 01 April 2008
- **J. COMLEY**. ELISA Assays: recent innovations take analyte detection to new levels.. *Drug Discovery World*, 2012
- **G. M. RAAB.** Comparison of a Logistic and a Mass-Action Curve for Radioimmunoassay Data. *Clin. Chem. CLIN. CHEM.*, 1983, vol. 29 (10), 1757-1761
- **D. FINNEY**. Response Curves for Radioimmunoassay.. *Clin. Chem.*, 1983, vol. 29 (10), 1762-1766
- **R. A. DUDLEY** ; **P. EDWARDS** ; **R. P. EKINS** ; **D. J. FINNEY** ; **I. G. M. MCKENZIE** ; **G. M. RAAB** ; **D. RODBARD** ; **R. P. C. RODGERS.** Guidelines for Immunoassay Data Processing. *Clin. Chem.*, 1985, vol. 31 (8), 1264-1271
- **P STROHNER** ; **D SARRACH** ; **J G. REICH**. Use of a modified binding model for the investigation of affinity dependence on antibody concentration in immunoassay systems.. *Journal of Immunological Methods*, 1997, vol. 203, 113-122
- **P. G. GOTTSCHALK** ; **J. R. DUNN**. The five-parameter logistic: A characterization and comparison with the four-parameter logistic.. *Analytical Biochemistry*, 2005, vol. 343, 54-65
- *FEBS Letters*, 1996, vol. 392, 245-249